# EUROPÄISCHE PATENTSCHRIFT

(11) **EP 1 484 106 B1**
(45) Veröffentlichungstag und Bekanntmachung des Hinweises auf die Patenterteilung: **10.01.2007**
(21) Anmeldenummer: 04011076.9
(22) Anmeldetag: 10.05.2004
(51) Int. Cl.: B01J 20/26

(54) **Fibrinogenadsorber III**
Fibrinogen Adsorber III
Adsorbeur de fibrinogène III

(30) Priorität: 04.06.2003 DE 10325304
(43) Veröffentlichungstag der Anmeldung: 08.12.2004
(73) Patentinhaber: Fresenius Medical Care Deutschland GmbH, 61352 Bad Homburg (DE)
(72) Erfinder: Metzger, Wolfgang, 66359 Bous (DE); Otto, Veit, Dr., 66606 St. Wendel (DE); Rüger, Walter, Dr., 36277 Schenklengsfeld (DE); Schimmel, Martin, Dr., 61206 Wöllstadt (DE)
(74) Vertreter: Müller-Boré & Partner Patentanwälte

(56) Entgegenhaltungen:
- DE-A- 2 429 191
- DE-A- 3 926 539
- DE-A- 10 011 482
- DE-A- 10 045 434
- PATENT ABSTRACTS OF JAPAN Bd. 2000, Nr. 20, 10. Juli 2001 (2001-07-10) & JP 2001 066649 A (COMMUNICATION RESEARCH LABORATORY MPT), 16. März 2001 (2001-03-16)

## Beschreibung

Die Erfindung betrifft ein Adsorbens zum Absenken der Konzentration von Fibrinogen und/oder Fibrin in Blut oder Blutplasma, umfassend eine organische Matrix mit kovalent an die Matrix gebundenen synthetischen Seitenketten, die endständige vicinale, durch Hydrolyse von Epoxygruppen gebildete Hydroxygruppen aufweisen, wobei die synthetischen Seitenketten Peptid-frei sind und keine aromatischen Gruppen aufweisen. Des weiteren betrifft die Erfindung ein Verfahren zur Herstellung des Adsorbens sowie die Verwendung des Adsorbens zur Herstellung eines Adsorbers zur Absenkung der Konzentration von Fibrinogen und/oder Fibrin in Blut oder Blutplasma.

Adsorbentien sind in der Medizintechnik weit verbreitet. Häufig beschrieben werden Adsorber mit Adsorbentien, die aus Blut Lipoproteine niedriger Dichte (LDL) entfernen bzw. deren Konzentration herabsetzen, wie dies aus der DE 39 32 971 bekannt ist. Diese Veröffentlichung beschreibt das Adsorbermaterial als einen organischen Träger mit festgelegter Partikelgröße und Ausschlußgrenze, der auf seiner Oberfläche einen Liganden trägt, an den das LDL-Molekül bindet.

In der DE 197 29 591 ist die Verwendung eines Liganden für Fibrinogen und/oder Fibrin beschrieben, um die aufgrund überhöhten Fibrinogenanteils im Blut erzeugten Krankheiten zu heilen oder diesen zumindest vorzubeugen. Der Ligand ist dabei in der DE 197 29 591 als eine Substanz definiert, die spezifisch an Fibrinogen und/oder Fibrin bindet und ist vorzugsweise ein Peptid mit drei bis 10 Aminosäuren.

Aus Artificial Organs, Band 20, Nr. 9 (1996), Seiten 986-990, ist die Reduzierung der Konzentrationen von Plasmafibrinogen, Immunoglobulin G (IgG) und Immunoglobulin M (IgM) durch Immunoadsorptionstherapie mit Tryptophan- oder Phenylalaninadsorbentien bekannt. In der Immunoadsorptionstherapie werden Adsorptionssäulen verwendet, die als Träger sphärische Polyvinylalkohol (PVA)-Gelteilchen aufweisen. Die PVA-Gelteilchen tragen auf ihrer Oberfläche entweder Tryptophan oder Phenylalanin als Aminosäureligand, der über Spacer kovalent an das PVA gebunden ist. Das von Blutzellen abgetrennte Plasma wird über die Adsorptionsäule geleitet und danach vor der Rückführung in den Patienten wieder mit den Blutzellen vereinigt. Mit dieser Immunoadsorptionstherapie werden gleichzeitig die Konzentrationen von Fibrinogen, IgG und IgM signifikant reduziert.

Auch wenn die Adsorption als Mittel zur Linderung von Krankheiten mittlerweile in den klinischen Alltag eingezogen ist, so werden doch steigende Anforderungen an die Selektivität der Adsorption gestellt. Das heißt, daß die Adsorber zum einen keine bzw. so wenig wie möglich für den Menschen notwendige Proteine adsorbieren dürfen, aber auf der anderen Seite die Herabsetzung der Konzentration schädlicher Proteine so hoch ist, daß die den Patienten belastende extrakorporale Behandlung möglichst effektiv wird.

Es ist seit einiger Zeit bekannt, daß eine Reihe von Krankheiten auf mangelnder Mikrozirkulation des Blutes beruhen. Als Beispiele seien die nachfolgend aufgeführten Krankheiten genannt.

ZNS: Schlaganfall, TIA (Transient Ischemic Attack), PRIND (Prolonged Reversible Ischemic Neurological Deficit), chronische vaskuläre Erkrankungen des ZNS, chronische intrakranielle Durchblutungsstörungen, chronische extrakranielle Durchblutungsstörungen, zerebrovaskuläre Durchblutungsstörungen, Demenz, Alzheimer Krankheit, schwerer zentraler Schindel
Auge: chronische Durchblutungsstörung, akuter Gefäßverschluß
Ohr: Hörsturz, Innenohr bedingter Schwindel, Morbus Menière
Lunge: primäre pulmonale Hypertonie, venooklusive Erkrankungen der Lunge, thrombotische primäre pulmonale Hypertonie, thromboembolische Erkrankungen der großen Gefäße
Herz: Transplantationsvaskulopathien, akuter Myokardinfarkt, instabile Angina pectoris, small vessel disease des Herzens, nicht operable schwere koronare Herzkrankheit, Kardiomyopathien
Abdomen: Angina abdominalis
Nieren: Vaskulopathien der Nieren, Glomerulonephritiden, chronische Niereninsuffizienz
Periphere arterielle Verschlußkrankheiten
   Akute Gefäßverschlüsse
   Vaskulitiden
   Septischer Schock
   Disseminierte intravaskuläre Koagulation (DIC) anderer Genese, z.B. bei Tumorerkrankungen
   Diabetes Typ I + II
   Diabetische Retinopathie
   Diabetische Neuropathie
   Diabetische Nephropathie

Bislang werden diese Krankheiten hauptsächlich medikamentös behandelt und dabei oft nur die Symptome beseitigt. Die bislang bekannten Maßnahmen, die Mikrozirkulation und die Rheologie des Blutes zu behandeln und zu beeinflussen, bestehen im Plasmaaustausch, der Heparin-induzierten extrakorporalen LDL-Cholesterin-Präzipitation (HELP) und in der Adsorption von Fibrinogen mit Hilfe eines Liganden, an den Fibrin und/oder Fibrinogen spezifisch bindet. Die Verwendung eines derartigen Liganden ist in der DE 197 29 591 beschrieben. Als Liganden werden Peptide genannt, die vorzugsweise 3 bis 10 Aminosäuren aufweisen, wobei die besonders bevorzugte Sequenz Glycin-Prolin-Arginin-Prolin-X sein soll.

Die synthetische Herstellung von Peptiden ist jedoch ein umständliches und kostenaufwendiges Verfahren, so daß der Einsatz als Ligand eines spezifischen Adsorbers sehr kostspielig ist. Darüber hinaus lösen Peptide ab einer bestimmten Länge bereits Antikörperreaktionen aus, so daß es nach wiederholter Anwendung langfristig zu heftigen Immunreaktionen kommen kann. Zwar werden, um die Immunabwehr herabzusetzen, möglichst kurze Peptidoligomere verwendet, doch kann eine Immunogenität nie voll ausgeschlossen werden. Außerdem ist eine Leckage, ein unbemerktes Ablösen von Peptidstücken, besonders gefährlich, da Peptide als Bestandteil von körpereigenen Strukturen bioaktive Moleküle darstellen.

Auch die Immunoadsorptionstherapie, wie in Artificial Organs, Band 20, Nr. 9 (1996), Seiten 986-990 beschrieben, verwendet die Aminosäuren Tryptophan oder Phenylalanin zur Anbindung an die PVA-Gelteilchen und ist daher ebenfalls umständlich und kostspielig. Überdies werden mit dieser Therapie auch Substanzen, die nicht aus dem Plasma entfernt werden sollen, wie IgG und IgM, in vergleichbaren Mengen aus dem Plasma abgetrennt, wie das Fibrinogen.

In EP-A1-1 132 128 und EP-A1-1 132 129 werden peptidfreie Adsorberbeads beschrieben. Es wurde jedoch festgestellt, daß die in EP-A1-1 132 128 und EP-A1-1 132 129 beschriebenen Adsorberbeads für eine praktische Anwendung eine zu starke Thrombozytenbindung zeigen. Für den Einsatz derartiger Adsorberbeads im Rahmen der Abreicherung von Fibrinogen in Vollblut oder Blutkomponenten sowie PRP (platelet rich plasma) bedarf es somit noch Verbesserungen, insbesondere hinsichtlich einer geringeren Affinität zu Thrombozyten bei gleichzeitig hoher Fibrinogenaffinität.

Somit liegt der vorliegenden Erfindung die Aufgabe zugrunde, ein Adsorbens zum Absenken der Konzentration von Fibrinogen und/oder Fibrin in Blut oder Blutplasma bereitzustellen, das hohe Eliminationsraten aufweist, einfach herzustellen und biokompatibel ist, keine Immunabwehr hervorruft und gegenüber den im Stand der Technik verfügbaren Adsorbentien eine geringere Affinität zu Thrombozyten bei gleichzeitig hoher Fibrinogenaffinität zeigen soll.

Diese Aufgabe wird durch die in den Ansprüchen gekennzeichneten Ausführungsformen gelöst.

Insbesondere betrifft die vorliegende Erfindung ein Adsorbens zum Absenken der Konzentration von Fibrinogen und/oder Fibrin in Blut oder Blutplasma, umfassend eine organische Matrix mit kovalent an die Matrix gebundenen synthetischen Seitenketten, die endständige vicinale, durch Hydrolyse von endständigen Epoxygruppen gebildete Hydroxygruppen aufweisen, wobei die synthetischen Seitenketten Peptid-frei sind und keine aromatischen Gruppen aufweisen.

Überraschenderweise hat sich herausgestellt, daß ein Adsorbens, welches eine organische Matrix mit kovalent an die Matrix gebundenen synthetischen Seitenketten, die endständige vicinale, durch Hydrolyse von endständigen Epoxygruppen gebildete Hydroxygruppen aufweisen, umfasst, eine deutliche Absenkung des Fibrinogenspiegels bewirkt, sodaß die Mikrozirkulation nach Behandlung verbessert ist, wobei das erfindungsgemäße Adsorbens gegenüber den im Stand der Technik verfügbaren Adsorbentien dabei gleichzeitig eine geringere Affinität zu Thrombozyten zeigt.

Wichtig für den Einsatz eines derartigen Adsorbens ist die Möglichkeit der Sterilisierbarkeit, insbesondere der Hitzesterilisierbarkeit, da das behandelte Blut wieder dem Patienten zugeführt werden soll und keine Sepsis oder Entzündungen auslösen darf. Im Gegensatz zu der erfindungsgemäßen Matrix, die eine organische Matrix ist und stabile Seitenketten aufweist, sind die im Stand der Technik verwendeten Peptide und Aminosäuren nicht hitzestabil oder chemisch stabil. Daher sind die kovalent an die Matrix gebundenen synthetischen Seitenketten vollständig frei von Peptiden.

Zudem hat sich erwiesen, daß die Anwesenheit von aromatischen Gruppen in den Seitenketten die Bindungskapazität ungünstig beeinflußt und auch die Selektivität des Absorbens hinsichtlich Fibrinogen und/oder Fibrin vermindert ist. Daher weisen die kovalent gebundenen synthetischen Seitenketten des erfindungsgemäßen Adsorbens keine aromatischen Gruppen auf.

Der hier gebrauchte Ausdruck "synthetisch" bedeutet, daß zur Einführung der Seitenketten in die Matrix kein biologisches Material verwendet wird, insbesondere keine Peptide, d.h. keine Dipeptide, Tripeptide, Oligopeptide, Polypeptide, Proteine (Makropeptide), selbst wenn sie synthetisch hergestellt worden sind. Beispielsweise kann die kovalent an die Matrix gebundene synthetische Seitenkette folgende Struktur aufweisen: 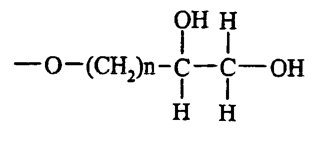 wobei n eine ganze Zahl im Bereich von 1 bis 18, vorzugsweise 1 bis 10, am meisten bevorzugt 1 ist.

Prinzipiell sind mehrere Materialien als organische Matrix möglich, wie beispielweise Kohlenhydrate oder organische Matrices, wie Copolymere von Acrylaten oder Methacrylaten sowie Polyamiden. Im Rahmen der vorliegenden Erfindung ist die organische Matrix vorzugsweise ein von (Meth)acrylsäureestern abgeleitetes Copolymer. Unter dem Begriff "(Meth)acryl" sind sowohl die entsprechenden Acryl- als auch Methacrylverbindungen zu verstehen. Mehr bevorzugt ist die organische Matrix ein Copolymer, abgeleitet von mindestens einem Epoxy(meth)acrylat und mindestens einem Vernetzer, ausgewählt aus der Gruppe, bestehend aus Alkylen-di(meth)acrylaten und Polyglykoldi(meth)acrylaten. Solche Copolymere werden vorzugsweise durch Suspensionspolymerisation hergestellt.

Als Matrix für das erfindungsgemäße Adsorbens ist ein durch Polymerisation der monomeren Einheiten
(i) Glycidylmethacrylat in einer Menge von 5 bis 95 Gew.-%, bevorzugt 40 bis 80 Gew.-%, am meisten bevorzugt 60 Gew.-%, und
(ii) Ethylenglykoldimethacrylat in einer Menge von 5 bis 95 Gew.-%, bevorzugt 20 bis 60 Gew.-%, am meisten bevorzugt 40 Gew.-%,
jeweils bezogen auf das Gesamtgewicht der monomeren Einheiten, hergestelltes, statistisches Copolymer am meisten bevorzugt.

Im Rahmen der vorliegenden Erfindung wird die Epoxygruppen (Oxirangruppen) enthaltende organische Matrix, wie z.B. das vorstehend angeführte Copolymer, erfindungsgemäß unter Bildung endständiger vicinaler Hydroxygruppen, d.h. 1,2-Diolgruppen, hydrolysiert. Die Hydrolyse kann beispielsweise durch Inkubation bei einer Temperatur im Bereich von etwa Raumtemperatur bis 90°C für eine Zeitdauer im Bereich von etwa 30 Minuten bis 24 Stunden in 1 bis 8 M NaOH, vorzugsweise 4 M NaOH, durchgeführt werden. Vorzugsweise weist die organische Matrix eine Hydroxyzahl im Bereich von 50 bis 1000 µmol/g, bezogen auf das Trockengewicht des Adsorbermaterials, auf. Die Hydroxyzahl läßt sich durch den Epoxygruppengehalt des eingesetzten Copolymers steuern.

Die Matrix kann in der Form von sphärischen, unaggregierten Partikeln, sog. Beads, Fasern oder einer Membran vorliegen, wobei eine Porosität der Matrix die Oberfläche erhöht. Die Porosität kann beispielsweise durch Zugabe von Porenbildnern, wie Cyclohexanol oder 1-Dodecanol, zu der Reaktionsmischung der Suspensionspolymerisation erreicht werden. Es ist des weiteren vorteilhaft, wenn die Matrix eine Ausschlußgrenze von mindestens 10⁷ Dalton besitzt, so daß das Fibrinogen mit dem Plasma in die Poren eindringen kann, um zu den endständige vicinale Hydroxygruppen enthaltenden Seitenketten der organischen Matrix zu gelangen.

Eine weitere vorteilhafte Ausgestaltung der Erfindung liegt darin, den erfindungsgemäßen Adsorber durch die geeignete Wahl der Trägermatrix im Vollblut einzusetzen. Dazu besteht die Matrix aus unaggregierten, sphärischen Partikeln, vorzugsweise solchen mit einer Partikelgrößenverteilung im Bereich von 50 bis 250 µm und einer Porenradiusverteilung im Bereich von 10 bis 200 nm. Dadurch können Blutzellen in Kontakt mit dem Adsorbermaterial treten, ohne daß sich die Säule zusetzt oder unzumutbar viele Zellen zurückgehalten werden oder aggregieren. Dies wird bei dem erfindungsgemäßen Adsorbens durch die Größe und die sphärische Gestalt der Beads ermöglicht, da die Zellen an der glatten äußeren Oberfläche der Beads entlanggleiten, wodurch nur geringe Thrombozytadhäsion erfolgt und das Plasma mit dem Fibrinogen dennoch die Möglichkeit hat, in die Poren einzudringen.

Dadurch entfallen extrakorporale Schritte, wie die Abtrennung von Blutzellen, das Behandeln des isolierten Plasmas und das Zusammenführen der Blutbestandteile, wodurch die Biokompatibilität des Verfahrens gesteigert wird, beispielsweise die Gefahr einer Komplementaktivierung weiter erheblich verringert wird. Das Entfallen extrakorporaler Schritte bewirkt eine Verkürzung der Behandlungszeit und eine Vereinfachung des Verfahrens, wodurch eine Erhöhung der Sicherheit und des Wohlbefindens des Patienten erreicht wird.

Ein mit dem erfindungsgemäßen Adsorbens ausgerüsteter Adsorber weist ein Gehäuse auf, welches vorzugsweise in Röhren- oder Säulenform ausgebildet ist, und welches das Adsorbens als Füllmaterial enthält. Im Hinblick auf die üblicherweise durchzusetzenden Blut- bzw. Blutplasmamengen und die Effizienz des erfindungsgemäßen Adsorbers umfaßt der Adsorber vorzugsweise ein Volumen von 250 bis 1250 ml. Der Adsorber kann einzeln oder im Doppel- oder Mehrfachbetrieb eingesetzt werden. Bei zwei oder mehr Adsorbern besteht die Möglichkeit, abwechselnd einen Adsorber mit dem Blut bzw. Blutplasma zu beschicken, während der andere Adsorber regeneriert wird. Dies führt zu einer weiteren Effizienz beim Einsatz des erfindungsgemäßen Adsorbers. Der Adsorber ist vorzugsweise so ausgebildet, daß er ein Gehäuse mit einem kopfseitigen Einlaßbereich aufweist, durch den das Blut oder Blutplasma dem Adsorber zugeführt wird, wobei sich in diesem Fall der Auslaß am Boden des Gehäuses des Adsorbers befindet.

Um zu verhindern, daß unerwünschte Substanzen, z.B. Substanzen, die vom Adsorbensmaterial herrühren, mit dem behandelten Blut bzw. Blutplasma in den Blutkreislauf des Patienten zurückgeführt werden, befindet sich am Auslaß des Gehäuses des Adsorbers vorzugsweise ein Filter. Dabei handelt es sich vorzugsweise um einen Partikelfilter.

Ein weiterer Gegenstand der vorliegenden Erfindung betrifft ein Verfahren zur Herstellung des vorstehenden Adsorbens zum Absenken der Konzentration von Fibrinogen und/oder Fibrin in Blut oder Blutplasma, umfassend die folgenden Schritte:
(a) Bereitstellen der organischen Matrix mit kovalent an die Matrix gebundenen synthetischen Seitenketten, die endständige Epoxygruppen aufweisen,
(b) Hydrolysieren der Epoxygruppen der organischen Matrix unter Einführung endständiger vicinaler Hydroxygruppen in die kovalent an die Matrix gebundenen synthetischen Seitenketten und
(c) gegebenenfalls Hitzebehandeln des in Schritt (b) erhaltenen Materials bei einer Temperatur von ≥ 100°C.

Durch ein derartiges Verfahren ist ein Adsorbermaterial erhältlich, das einfach herzustellen und biokompatibel ist und keine Immunabwehr hervorruft. Überraschend ist, daß die einfach durchzuführende Hydrolyse der organischen Matrix mit kovalent an die Matrix gebundenen synthetischen Seitenketten, die endständige Epoxygruppen aufweisen, wie vorstehend beschrieben, zu einem Adsorbermaterial mit einer ausgezeichneten Fibrinogenbindungskapazität bei gleichzeitig verminderter Affinität zu Thrombozyten führt.

Wichtig für den Einsatz eines derartigen Adsorbens ist die Möglichkeit der Sterilisierbarkeit, insbesondere der Hitzesterilisierbarkeit bei einer Temperatur im Bereich von vorzugsweise 100 bis 140 °C, mehr bevorzugt 121°C, für eine Zeitdauer von beispielsweise 20 min bis 60 min, da das behandelte Blut wieder dem Patienten zugeführt werden soll und keine Sepsis oder Entzündungen auslösen darf. Dabei ist besonders vorteilhaft, daß mit einem einzigen Verfahrensschritt die Hitzebehandlung und die Sterilisierbarkeit gleichzeitig durchgeführt werden können. Darüber hinaus hat sich das erfindungsgemäß erhältliche Adsorbens als biokompatibel erwiesen.

Die in dem erfindungsgemäßen Verfahren verwendete organische Matrix weist vorkann insbesondere durch eine Suspensionspolymerisation hergestellt werden, wie beispielsweise in WO 95/26988 beschrieben.

Als Matrix wird im Rahmen des erfindungsgemäßen Verfahrens am meisten bevorzugt ein durch Polymerisation der monomeren Einheiten
(i) Glycidylmethacrylat in einer Menge von 5 bis 95 Gew.-%, bevorzugt 40 bis 80 Gew.-%, am meisten bevorzugt 60 Gew.-%, und
(ii) Ethylenglykoldimethacrylat in einer Menge von 5 bis 95 Gew.-%, bevorzugt 20 bis 60 Gew.-%, am meisten bevorzugt 40 Gew.-%,
jeweils bezogen auf das Gesamtgewicht der monomeren Einheiten, hergestelltes, statistisches Copolymer eingesetzt.

Die Hydrolyse im Rahmen des erfindungsgemäßen Verfahrens kann beispielsweise durch Inkubation bei einer Temperatur im Bereich von etwa Raumtemperatur bis 90°C für eine Zeitdauer im Bereich von etwa 30 Minuten bis 24 Stunden in 1 bis 8 M NaOH, vorzugsweise 4 M NaOH, durchgeführt werden.

### Beispiel

Es wurde ein Copolymer aus Ethylenglycoldimethacrylat (EGDMA) und Glycidylmethacrylat (GMA) durch Suspensionspolymerisation gemäß dem in WO 95/26988 beschriebenen Verfahren hergestellt. Ein Gemisch aus 181 g EGDMA und 272 g GMA und den Lösungsmitteln Cyclohexanol (542 g) und Dodecanol (54 g) und dem Initiator AIBN (1 Gew.-% bezogen auf die Gesamtheit der Monomerbausteine) wurde unter Rühren in 3075 ml einer Polyvinylalkohol-Lösung in Wasser gegeben. Das Gemisch wurde bei 54°C für 2 h polymerisiert. Nach Auspolymerisieren der Restmonomere bei 75°C und 88°C wurde das erhaltene Material mit Isopropanol und Wasser gewaschen und fraktioniert.

unter Rühren in 3075 ml einer Polyvinylalkohol-Lösung in Wasser gegeben. Das Gemisch wurde bei 54°C für 2 h polymerisiert. Nach Auspolymerisieren der Restmonomere bei 75°C und 88°C wurde das erhaltene Material mit Isopropanol und Wasser gewaschen und fraktioniert.

Das derart erhaltene Material wurde bei einer Temperatur von etwa 70°C für eine Zeitdauer von 30 Minuten der Hydrolyse mit 4 M NaOH unterworfen.

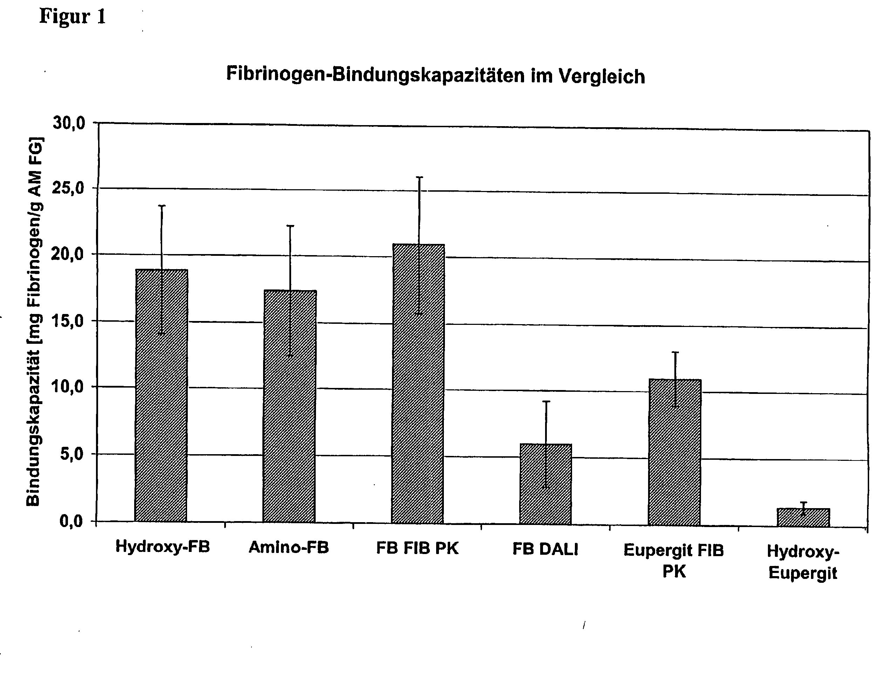

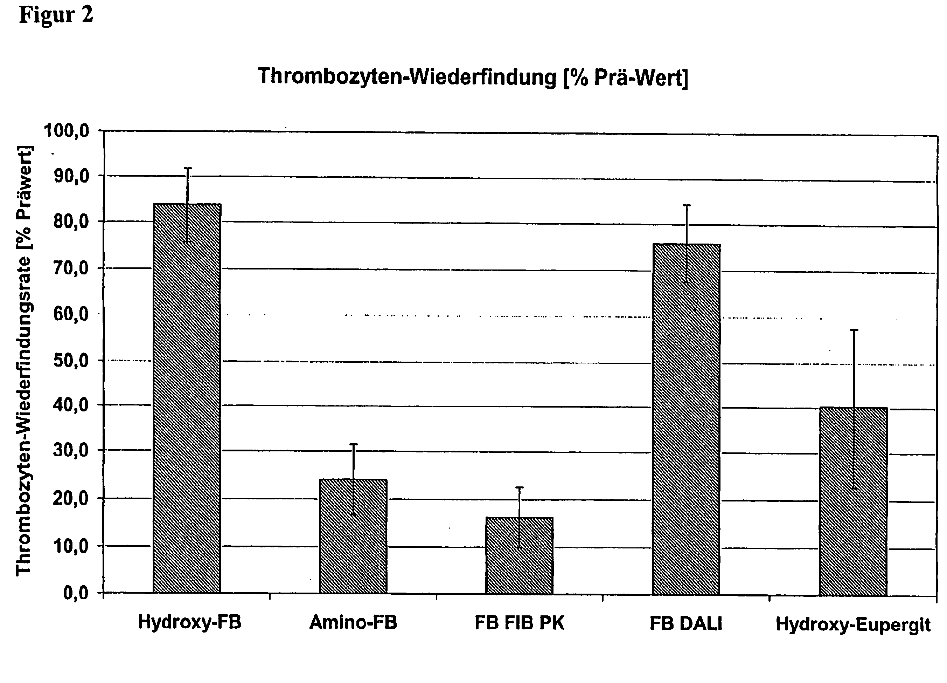

5 ml hitzesterilisiertes Adsorbermaterial (AM) wurde in kleine Chromatographiesäulen eingefüllt. Die Vorbehandlung des Adsorbens mit 80 ml Elektrolytlösung (Primer-Lösung) wurde mit einem Fluss von 5,2 ml/min über eine Schlauchpumpe durchgeführt. 30 ml Vollblut (15:1 mit Citrat antikoaguliert) wurden nachfolgend mit einem Fluss von 3,25 ml/min über die Säule prozessiert. Es wurden jeweils sechs Fraktionen ä 5 ml gesammelt. Die Fibrinogenkonzentrationen im Präwert und in den einzelnen Fraktionen wurden mit der Methode nach CLAUSS (Clauss, A., Gerinnungsphysiologische Schnellmethode zur Bestimmung des Fibrinogens: Acta Haematologica (1957) 17, 237-246), an dem Koagulometer Thrombotimer 4 (Behnk-Elektronik, Norderstedt) bestimmt. Die Bindungskapazität ergibt sich aus der Differenz der Prä- und gemittelten Postwerte und wurde als pro Gramm AM Feuchtgewicht (FG) absolut gebundenes Fibrinogen [mg] angegeben (siehe Figur 1). Die Thrombozytenwiederfindungsrate wurde durch Bestimmung der Blutbilder von Präwert und den einzelnen Fraktionen im Zellanalysator Sysmex K-1000 (Fa. Sysmex) bestimmt und als prozentuale Absenkung gegenüber dem Präwert angegeben (siehe Figur 2). Der Versuch wurde mit acht verschiedenen Blutspendern durchgeführt. Die bestimmten Fibrinogen-Präwerte lagen im Mittel bei 328 mg/dl ± 78 mg/dl.

In der Fig. 1 ist die Fibrinogenabsenkung, d.h. die Fibrinogenbindung in mg bezogen auf g AM FG, im Vergleich zu bestimmten Referenz- bzw. Vergleichsadsorbentien gezeigt. In der Fig. 2 ist die Thrombozyten-Wiederfindungsrate bzw. Thrombozyten-Recovery, bezogen auf % Präwert, im Vergleich zu den entsprechend gewählten Referenz- bzw Vergleichsadsorbentien gezeigt. Dabei sind:
- Hydroxy-FB:: erfindungsgemäßes Adsorbens gemäß vorstehendem Beispiel
- Amino-FB:: Adsorbens gemäß Beispiel 2 von EP-A1-1 132 129
- FB FIB PK:: Adsorbens gemäß EP-A1-1 132 128 mit organischer Matrix auf Basis von Glycidylmethacrylat-Ethylenglykoldimethacrylat-Copolymer
- FB Dali: Adsorbens gemäß Beispiel 1 von EP-A1-0 424 698
- Eupergit FIB PK:: Adsorbens gemäß EP-A1-1 132 128 mit organischer Matrix auf Basis von Eupergit, vertrieben von Röhm GmbH & Co. KG, Darmstadt
- Hydroxy-Eupergit:: NaOH-Hydrolysat von Eupergit

Aus der Figur 1 ist ersichtlich, daß das erfindungsgemäße Adsorbens Fibrinogen nahezu genauso stark bindet wie das in EP-A1-1 132 129 bzw. EP-1 132 128 beschriebene aminierte Trägermaterial. Ferner ist aus Figur 1 ersichtlich, daß das erfindungsgemäße Adsorbens Fibrinogen deutlich stärker bindet als beispielsweise das Hydrolysat von Eupergit, vertrieben von Röhm GmbH & Co. KG, Darmstadt. Der überraschende Effekt der vorliegenden Erfindung ist aus Figur 2 ersichtlich. So zeigt das erfindungsgemäße Adsorbens, welches Fibrinogen nahezu genauso stark bindet wie das in EP-A1-1 132 129 bzw. EP-A1-132 128 beschriebene aminierte Trägermaterial, gegenüber diesen eine drastisch gesteigerte Thrombozyten-Recovery. Das erfindungsgemäße Adsorbens zeichnet sich somit überraschenderweise durch eine deutliche Absenkung des Fibrinogenspiegels bei gleichzeitig geringer Affinität zu Thrombozyten aus und ist dadurch den vorstehend aufgeführten Referenz- bzw. Vergleichsadsorbentien in dieser Eigenschaftsbalance überlegen.

Ohne daran gebunden zu sein, könnte eine Erklärung bezüglich dieses überraschenden Effekts der erfindungsgemäßen Adsorbentien darauf beruhen, daß sich die Wechselwirkung von Fibrinogen mit Thrombozyten über die Wechselwirkung von GP IIb/IIIA (thrombozytäres Membranglykoprotein) mit Integrin-Bindungssequenzen der D-Domäne des Fibrinogenmoleküls vollzieht. Je nach Bindungsstärke von Fibrinogen an unphysiologische Oberflächen, die mit steigendem Hydrophobie-Grad zunimmt, kann sich die Konformation der D-Domäne ändern, was dazu führt, daß Thrombozyten nicht mehr wechselwirken können. Die erfindungsgemäßen Adsorbentien kombinieren also hohe Fibrinogenbindung mit hoher Thrombozyten-Recovery und können somit vorteilhaft auch zur Vollblutanwendung vorgesehen werden.

## Patentansprüche

1. Adsorbens zum Absenken der Konzentration von Fibrinogen und/oder Fibrin in Blut oder Blutplasma, umfassend eine organische Matrix mit kovalent an die Matrix gebundenen synthetischen Seitenketten, die endständige vicinale, durch Hydrolyse von endständigen Epoxygruppen gebildete Hydroxygruppen aufweisen, wobei die synthetischen Seitenketten Peptid-frei sind und keine aromatischen Gruppen aufweisen.

2. Adsorbens nach Anspruch 1, wobei die organische Matrix ein von (Meth)acrylsäureestem abgeleitetes Copolymer ist.

3. Adsorbens nach Anspruch 1 oder 2, wobei die organische Matrix eine Hydroxyzahl im Bereich von 50 bis 1000 µmol/g, bezogen auf das Trockengewicht des Adsorbermaterials, aufweist.

4. Adsorbens nach einem oder mehreren der vorhergehenden Ansprüche, wobei die organische Matrix ein Copolymer, abgeleitet von mindestens einem Epoxy(meth)acrylat und mindestens einem Vernetzer, ausgewählt aus der Gruppe, bestehend aus Alkylendi(meth)acrylaten und Polyglykoldi(meth)acrylaten, ist.

5. Adsorbens nach Anspruch 4, wobei das Copolymer ein durch Polymerisation der monomeren Einheiten
(i) Glycidylmethacrylat in einer Menge von 5 bis 95 Gew.-%, bevorzugt 40 bis 80 Gew.-%, am meisten bevorzugt 60 Gew.-%, und
(ii) Ethylenglykoldimethacrylat in einer Menge von 5 bis 95 Gew.-%, bevorzugt 20 bis 60 Gew.-%, am meisten bevorzugt 40 Gew.-%, jeweils bezogen auf das Gesamtgewicht der monomeren Einheiten, hergestelltes, statistisches Copolymer ist.

6. Adsorbens nach einem oder mehreren der vorhergehenden Ansprüche, wobei die organische Matrix aus sphärischen, unaggregierten Partikeln aufgebaut ist.

7. Adsorbens nach einem oder mehreren der vorhergehenden Ansprüche, wobei die organische Matrix aus sphärischen, unaggregierten Partikeln mit einer Partikelgrößenverteilung von 50 bis 250 µm aufgebaut ist.

8. Adsorbens nach einem oder mehreren der vorhergehenden Ansprüche, wobei die organische Matrix aus porösen, sphärischen, unaggregierten Partikeln mit einer Partikelgrößenverteilung von 50 bis 250 µm und einer Porenradiusverteilung im Bereich von 10 bis 200 nm aufgebaut ist.

9. Verwendung des Adsorbens nach einem oder mehreren der Ansprüche 1 bis 8 zur Herstellung eines Adsorbers zur Absenkung der Konzentration von Fibrinogen und/oder Fibrin in Blut oder Blutplasma.

10. Verfahren zum Herstellen des Adsorbens nach einem oder mehreren der Ansprüche 1 bis 8, umfassend die folgenden Schritte:
(a) Bereitstellen der organischen Matrix mit kovalent an die Matrix gebundenen synthetischen Seitenketten, die endständige Epoxygruppen aufweisen,
(b) Hydrolysieren der Epoxygruppen der organischen Matrix unter Einführung endständiger vicinaler Hydroxygruppen in die kovalent an die Matrix gebundenen synthetischen Seitenketten und
(c) gegebenenfalls Hitzebehandeln des in Schritt (b) erhaltenen Materials bei einer Temperatur von ≥ 100°C.

11. Verfahren nach 10, wobei die in Schritt (a) bereitgestellte organische Matrix einen Epoxygruppengehalt von 25 bis 500 µmol/g, bezogen auf das Trokkengewicht des Adsorbermaterials, aufweist.

12. Verfahren nach Anspruch 10 oder 11, wobei die organische Matrix ein Copolymer, abgeleitet von mindestens einem Epoxy(meth)acrylat und mindestens einem Vernetzer, ausgewählt aus der Gruppe, bestehend aus Alkylendi(meth)acrylaten und Polyglykoldi(meth)acrylaten, ist.

13. Verfahren nach Anspruch 12, wobei das Copolymer durch eine Suspensionspolymerisation hergestellt wird.

14. Verfahren nach Anspruch 12 oder 13, wobei das Copolymer ein durch Polymerisation der monomeren Einheiten
(i) Glycidylmethacrylat in einer Menge von 5 bis 95 Gew.-%, bevorzugt 40 bis 80 Gew.-%, am meisten bevorzugt 60 Gew.-%, und
(ii) Ethylenglykoldimethacrylat in einer Menge von 5 bis 95 Gew.-%, bevorzugt 20 bis 60 Gew.-%, am meisten bevorzugt 40 Gew.-%,
jeweils bezogen auf das Gesamtgewicht der monomeren Einheiten, hergestelltes, statistisches Copolymer ist.

15. Verfahren nach einem oder mehreren der Ansprüche 10 bis 14, worin die Hydrolyse durch Inkubation bei einer Temperatur im Bereich von Raumtemperatur bis 90°C für eine Zeitdauer im Bereich von 30 Minuten bis 24 Stunden in 1 bis 8 M NaOH, vorzugsweise 4 M NaOH, durchgeführt wird.

16. Verfahren nach einem oder mehreren der Ansprüche 10 bis 15, wobei die Hitzebehandlung eine Sterilisationsbehandlung bei einer Temperatur im Bereich von 100 bis 140 °C ist.

## Claims

1. Adsorbent for lowering the concentration of fibrinogen and/or fibrin in the blood or blood plasma, encompassing an organic matrix with synthetic side chains covalently bound to the matrix and exhibiting terminal vicinal hydroxy groups formed by the hydrolysis of terminal epoxy groups, said synthetic side chains being free of peptides and devoid of aromatic groups.

2. Adsorbent according to claim 1, in which the organic matrix is a copolymer derived from (meth)acrylic acid esters.

3. Adsorbent according to claim 1 or 2, in which the organic matrix has a hydroxyl number in the range from 50 to 1000 µmol/g as related to the dry weight of the adsorber material.

4. Adsorbent according to one or more of the preceding claims, in which the organic matrix is a copolymer derived from at least one epoxy(meth)acrylate and at least one cross-linking agent selected from the group comprising alkylene di(meth)acrylates and polyglycol di(meth)acrylates.

5. Adsorbent according to claim 4, in which the copolymer is a random copolymer produced by the polymerization of the monomeric units
(i) glycidyl methacrylate in an amount of 5 to 95 weight per cent, preferably 40 to 80 weight per cent and most preferably 60 weight per cent; and
(ii) ethylene glycoldimethacrylate in an amount of 5 to 95 weight per cent, preferably 20 to 60 weight per cent and most preferably 40 weight per cent,
relative in each case to the total weight of the monomeric units.

6. Adsorbent according to one or more of the preceding claims, in which the organic matrix is composed of spherical, nonaggregated particles.

7. Adsorbent according to one or more of the preceding claims, in which the organic matrix is composed of spherical, nonaggregated particles with a particle size distribution from 50 to 250 µm.

8. Adsorbent according to one or more of the preceding claims, in which the organic matrix is composed of porous, spherical, nonaggregated particles with a particle size distribution from 50 to 250 µm and a pore radius distribution in the range from 10 to 200 nm.

9. Use of the adsorbent according to one or more of claims 1 to 8 for producing an adsorber serving to lower the concentration of fibrinogen and/or fibrin in the blood or blood plasma.

10. Method for producing the adsorbent according to one or more of claims 1 to 8, comprising the following steps:
(a) Provision of the organic matrix having synthetic side chains covalently bound to the matrix and exhibiting terminal epoxy groups;
(b) Hydrolysis of the epoxy groups of the organic matrix while introducing terminal vicinal hydroxy groups into the synthetic side chains covalently bound to the matrix; and
(c) as required, heat treatment of the material obtained in step (b) at a temperature of ≥100°C.

11. Method according to claim 10, wherein the organic matrix provided in step (a) contains epoxy groups in the amount of 25 to 500 µmol/g as related to the dry weight of the adsorber material.

12. Method according to claim 10 or 11, wherein the organic matrix is a copolymer derived from at least one epoxy(meth)acrylate and at least one cross-linking agent selected from the group comprising alkylene di(meth)acrylates and polyglycol di(meth)acrylates.

13. Method according to claim 12, wherein the copolymer is produced by a suspension polymerization.

14. Method according to claim 12 or 13, wherein the copolymer is a random copolymer produced by the polymerization of the monomeric units
(i) glycidyl methacrylate in an amount of 5 to 95 weight per cent, preferably 40 to 80 weight per cent, and most preferably 60 weight per cent, and
(ii) ethylene glycol dimethacrylate in an amount from 5 to 95 weight per cent, preferably 20 to 60 weight per cent and most preferably 40 weight per cent,
relative in each case to the total weight of the monomeric units.

15. Method according to one or more of claims 10 to 14, wherein the hydrolysis is carried out by incubation, at a temperature in the range from room temperature to 90°C for a duration ranging from 30 minutes to 24 hours, in 1 to 8 M NaOH and preferably 4 M NaOH.

16. Method according to one or more of claims 10 to 15, wherein the heat treatment involves sterilization at a temperature in the range from 100 to 140°C.

## Revendications

1. Adsorbant pour abaisser la concentration de fibrinogène et/ou de fibrine dans le sang ou le plasma sanguin, comprenant une matrice organique avec des chaînes latérales synthétiques liées de manière covalente à la matrice, qui présentent des groupes hydroxy vicinaux terminaux formés par hydrolyse de groupes époxy terminaux, les chaînes latérales synthétiques étant sans peptides et présentant aucun groupe aromatique.

2. Adsorbant selon la revendication 1, dans lequel la matrice organique est un copolymère dérivé d'esters de l'acide (méth)acrylique.

3. Adsorbant selon la revendication 1 ou 2, dans lequel la matrice organique présente un indice hydroxy dans la plage allant de 50 à 1000 µmoles/g en se basant sur le poids à sec de la substance adsorbante.

4. Adsorbant selon l'une ou plusieurs des revendications précédentes, dans lequel la matrice organique est un copolymère, dérivé d'au moins un (méth)acrylate d'époxy et d'au moins un agent de réticulation, choisi dans le groupe constitué par des di(méth)acrylates d'alkylène et des di(méth)acrylates de polyglycol.

5. Adsorbant selon la revendication 4, dans lequel le copolymère est un copolymère statistique préparé par polymérisation des motifs monomères:
(i) méthacrylate de glycidyle dans une quantité de 5 à 95 % en poids, de préférence de 40 à 80 % en poids, de manière préférée entre toutes de 60 % en poids, et
(ii) diméthacrylate d'éthylène glycol dans une quantité de 5 à 95 % en poids, de préférence de 20 à 60 % en poids, de manière préférée entre toutes de 40 % en poids, respectivement, en se basant sur le poids total des motifs monomères.

6. Adsorbant selon l'une ou plusieurs des revendications précédentes, dans lequel la matrice organique est constituée par des particules sphériques non agrégées.

7. Adsorbant selon l'une ou plusieurs des revendications précédentes, dans lequel la matrice organique est constituée par des particules sphériques non agrégées avec une répartition de grosseur des particules allant de 50 à 250 µm.

8. Adsorbant selon l'une ou plusieurs des revendications précédentes, dans lequel la matrice organique est constituée par des particules poreuses, sphériques et non agrégées avec une répartition de grosseur des particules allant de 50 à 250 µm et une répartition du rayon des pores dans la plage allant de 10 à 200 nm.

9. Utilisation de l'adsorbant selon l'une ou plusieurs des revendications 1 à 8, pour préparer un adsorbant pour abaisser la concentration de fibrinogène et/ou de fibrine dans le sang ou dans le plasma sanguin.

10. Procédé pour préparer l'adsorbant selon l'une ou plusieurs des revendications 1 à 8, comprenant les étapes suivantes :
(a) la préparation de la matrice organique avec des chaînes latérales synthétiques liées de manière covalente à la matrice qui présentent des groupes époxy terminaux,
(b) l'hydrolyse des groupes époxy de la matrice organique lors de l'introduction de groupes hydroxy vicinaux terminaux dans les chaînes latérales synthétiques liées de manière covalente à la matrice et
(c) le cas échéant, le traitement thermique de la substance obtenue à l'étape (b) à une température supérieure ou égale à 100 °C.

11. Procédé selon la revendication 10, dans lequel la matrice organique préparée à l'étape (a) présente une teneur en groupes époxy de 25 à 500 µmoles/g en se basant sur le poids à sec de la substance adsorbante.

12. Procédé selon la revendication 10 ou 11, dans lequel la matrice organique est un copolymère dérivé d'au moins un (méth)acrylate d'époxy et d'au moins un agent de réticulation, choisi dans le groupe constitué par des di(méth)acrylates d'alkylène et des di(méth)acrylates de polyglycol.

13. Procédé selon la revendication 12, dans lequel le copolymère est préparé au moyen d'une polymérisation en suspension.

14. Procédé selon la revendication 12 ou 13, dans lequel le copolymère est un copolymère statistique préparé par polymérisation des motifs monomères :
(i) méthacrylate de glycidyle dans une quantité de 5 à 95 % en poids, de préférence de 40 à 80 % en poids, de manière préférée entre toutes de 60 % en poids, et
(ii) diméthacrylate d'éthylène glycol dans une quantité de 5 à 95 % en poids, de préférence de 20 à 60 % en poids, de manière préférée entre toutes de 40 % en poids, respectivement, en se basant sur le poids total des motifs monomères.

15. Procédé selon l'une ou plusieurs des revendications 10 à 14, dans lequel l'hydrolyse est effectuée par incubation à une température dans la plage allant de la température ambiante à 90 °C pour une durée dans la plage allant de 30 minutes à 24 heures dans 1 à 8 M de NaOH, de préférence dans 4 M de NaOH.

16. Procédé selon l'une ou plusieurs des revendications 10 à 15, dans lequel le traitement thermique est un traitement par stérilisation à une température dans la plage allant de 100 à 140°C.
